# EUROPEAN PATENT APPLICATION

(11) **EP 4 252 720 A1**
(43) Date of publication of application: **04.10.2023**
(21) Application number: 21896667.9
(22) Date of filing: 22.10.2021
(51) Int. Cl.: A61F 2/966

(54) **DELIVERY DEVICE**

(30) Priority: 26.11.2020 CN 202011348776
(71) Applicant: Lifetech Scientific (Shenzhen) Co., Ltd., Shenzhen, Guangdong 518063 (CN)
(72) Inventor: LIU, Caiping, Shenzhen, Guangdong 518052 (CN); LIU, Kui, Shenzhen, Guangdong 518052 (CN); XIA, Yunchen, Shenzhen, Guangdong 518052 (CN)
(74) Representative: dompatent von Kreisler Selting Werner - Partnerschaft von Patent- und Rechtsanwälten mbB
(86) International application number: PCT/CN2021/125607
(87) International publication number: WO 2022/111165

(57) **Abstract**

A delivery device (100) is provided, including: a sheath core (20); a TIP head (40), the TIP head (40) being arranged at a distal end of the sheath core (20); and a fixing anchor (30), the fixing anchor (30) is arranged on an outer wall of the sheath core (20) and close to a proximal end of the TIP head (40); the sheath core (20) is fixed relative to the TIP head (40); the fixing anchor (30) is provided with a mounting portion (34), and a pull wire (60) can be accommodated in the mounting portion (34). In a process of loading a stent, the pull wire (60) applies an acting force on the fixing anchor (30), and the fixing anchor (30) is fixedly connected to the sheath core (20) and thus drives the sheath core (20), the TIP head (40) and the stent to move synchronously. Since the pull wire (60) does not directly come into contact with the stent, the stent can be prevented from being deformed due to the pressing of the pull wire (60) on the stent. In addition, the stent is loaded by means of the pull wire (60), which prevents damage to a stent caused by pushing a sheath tube (10) of the prior art. Moreover, the stent will not move in the loading process, which prevents the stent from being separated from the fixing anchor (30).

## Description

### Technical Field

The embodiments relate to the technical field of medical instruments, and in particular to a delivery device.

### Background

As a new means for treating cardiovascular diseases, stents have been used more and more in clinical practice. A delivery device plays an important role in loading the stent and building intravascular channels.

Before use, the stent needs to be loaded into a sheath tube of the delivery device. In a common loading method, a proximal end of the stent is hung with a fixing anchor, and then a sheath core is pushed forward towards a distal end of the delivery device to push the stent into the sheath tube. Alternatively, a pull wire is threaded through the stent or wound around the sheath core at the proximal end of the fixing anchor, and the pull wire is pulled towards the distal end of the delivery device for auxiliary assembly.

However, when the stent is pushed forward into the sheath tube by taking the sheath core alone as a force applicator without the pull wire, the sheath core will be bent or broken under a force. However, it is very likely to cause the stent to be separated from the fixing anchor or cause the proximal end of the stent to deform in the pulling process of the pull wire assistance method.

### Summary

The embodiments aim to at least solve the problem of how to avoid separation or damage of a stent during loading of the stent. The objective is realized by the following technical solutions:
The embodiments provide a delivery device, including a sheath core; a TIP head, the TIP head arranged at a distal end of the sheath core; and a fixing anchor, wherein the fixing anchor is arranged on an outer wall of the sheath core and close to a proximal end of the TIP head; the sheath core is fixed relative to the TIP head; the fixing anchor is provided with a mounting portion, and a pull wire can be accommodated in the mounting portion.

In one embodiment, the fixing anchor includes: a fixing portion, which is arranged on an outer side of the sheath core and is fixed with the sheath core; an anchoring portion, which is configured for anchoring a stent; and a basic portion, wherein the fixing portion is connected with the anchoring portion through the basic portion, and the mounting position is arranged on the basic portion or the anchoring portion.

In one embodiment, the mounting position includes a cutting slot formed in the basic portion, and an opening of the cutting slot faces a proximal end of the basic portion; or the mounting position includes a through hole formed in the basic portion, and the through hole extends along an axial direction of the sheath core and axially extends through the basic portion; or the mounting position includes a via hole formed in the basic portion, and the via hole extends along a direction parallel to a radial direction of the sheath core and extends through the basic portion along the direction.

In one embodiment, the anchoring portion includes a plurality of rod-shaped members; the rod-shaped members are arranged at a distal end of the basic portion and extend towards the distal end; the mounting position includes punch holes formed in the rod-shaped members; and the punch holes extend along an axial direction of the sheath core.

In one embodiment, two punch holes are formed, and are arranged on opposite, adjacent or spaced rod-shaped members.

In one embodiment, the TIP head includes a cylindrical section; the cylindrical section is provided with a fixing position arranged along a longitudinal direction of the cylindrical section; and the pull wire can be accommodated in the fixing position.

In one embodiment, the TIP head further includes a first tapered section and a second tapered section; the first tapered section is coaxially arranged at a distal end of the cylindrical section; the second tapered section is coaxially arranged at a proximal end of the cylindrical section and is connected with the sheath core; the fixing position includes a via hole which is arranged along a longitudinal direction of the cylindrical section and axially extends through the cylindrical section; a port of a distal end of the via hole is located at a junction between the first tapered section and the cylindrical section; and a port of a proximal end of the via hole is located at a junction between the second tapered section and the cylindrical section.

In one embodiment, the TIP head further includes a first tapered section and a second tapered section; the first tapered section is coaxially arranged at a distal end of the cylindrical section; the second tapered section is coaxially arranged at a proximal end of the cylindrical section and is connected with the sheath core; the fixing position includes a groove which is arranged along a lengthwise direction of the cylindrical section; an opening of the groove is located on an outer surface of the cylindrical section; a distal end of the groove is located at a junction between the first tapered section and the cylindrical section; and a proximal end of the groove is located at a junction between the second tapered section and the cylindrical section.

In one embodiment, the delivery device further includes a fixing member, which surrounds a proximal end portion of the groove to fix a pull wire.

In one embodiment, a circumferential groove is arranged at a proximal end portion of the cylindrical section; and the fixing member can be accommodated in the circumferential groove, so that an outer surface of the fixing member is flush with an outer surface of the cylindrical section.

In summary, implementing the delivery device of the present embodiments have the following beneficial effects:
When a stent is loaded, the pull wire is threaded on the mounting position of the fixing anchor. The fixing anchor is pulled to drive the stent to enter the sheath tube, thus avoiding deformation of the stent due to the pushing of the sheath tube. In addition, in the process of pulling the pull wire, the pull wire directly applies an acting force to the fixing anchor, and the force is indirectly transmitted to the stent, preventing the pull wire from damaging the stent.

### Brief Description of the Drawings

By reading the detailed description in the preferred specific implementation modes below, various other advantages and benefits will become clear to those of ordinary skill in the art. The accompanying drawings are only used for the purpose of illustrating the preferred implementation modes, and are not considered as a limitation to the embodiments. Furthermore, throughout the drawings, the same reference numerals are used to denote the same components. In the drawings:
Fig. 1 is a schematic structural diagram of a delivery device according to the present invention;
Fig. 2 is a partial schematic structural diagram of the delivery device shown in Fig. 1;
Fig. 3 is a schematic structural diagram of a first implementation of a fixing anchor of the delivery device shown in Fig. 2;
Fig. 4 is a schematic structural diagram of a second implementation of the fixing anchor of the delivery device shown in Fig. 2;
Fig. 5 is a schematic structural diagram of a third implementation of the fixing anchor of the delivery device shown in Fig. 2;
Fig. 6 is a sectional view of the fixing anchor shown in Fig. 5 taken along line A-A thereof;
Fig. 7 is a schematic structural diagram of a fourth implementation of the fixing anchor of the delivery device shown in Fig. 2;
Fig. 8 is a sectional view of the fixing anchor shown in Fig. 7 taken along the line B-B thereof;
Fig. 9 is a schematic structural diagram showing the cooperation between the fixing anchor and a pull wire shown in Fig. 7;
Fig. 10 is a sectional view of the fixing anchor shown in Fig. 9 taken along line C-C thereof;
Fig. 11 is a schematic structural diagram of the fixing anchor from another viewing angle shown in Fig. 9;
Fig. 12 is a schematic structural diagram of a fifth implementation of the fixing anchor of the delivery device shown in Fig. 2;
Fig. 13 is a sectional view of the fixing anchor shown in Fig. 12 taken along line D-D thereof;
Fig. 14 is a schematic structural diagram of a first implementation of a TIP head of the delivery device shown in Fig. 2;
Fig. 15 is a partial sectional view of the TIP head shown in Fig. 14;
Fig. 16 is a radial sectional view of the TIP head shown in Fig. 14;
Fig. 17 is a schematic structural diagram showing the threading of a pull wire by the fixing anchor of the fourth implementation and the TIP head of the first implementation; and
Fig. 18 is a schematic structural diagram of a second implementation of a TIP head of the delivery device shown in Fig. 2.

Reference numerals in the drawings:
100: delivery device
10: sheath tube;
20: sheath core;
30: fixing anchor; 31: fixing portion; 32: basic portion; 33: anchoring portion; 34: mounting position;
40: TIP head; 41: first tapered section; 42: cylindrical section; 43: second tapered section; 44: fixing position;
50: handle;
60: pull wire;
70: fixing member.

### Detailed Description of the Embodiments

In order to make the foregoing objectives, features and advantages of the embodiments more obvious and understandable, the specific implementation modes of the embodiments are described in detail with reference to the accompanying drawings. Many specific details are described in the following descriptions to facilitate full understanding of the embodiments. However, the embodiments can be implemented in a variety of other ways than those described herein, and those skilled in the art can make similar improvements without departing from the connotation of the embodiments. Therefore, the embodiments are not limited by specific implementations disclosed below.

Unless otherwise defined, all technical and scientific terms used herein are the same as meanings of general understandings of those skilled in the art of the embodiments. The terms used in the description of the embodiments herein are merely to describe the specific implementation modes, not intended to limit the embodiments. The term "and/or" used herein includes any and all combinations of one or more related listed items.

It should be noted that when an element is referred to as being "fixed" or "arranged" to another element, it can be directly on the other element or an intermediate element may also exist. When one element is considered to be "connected" to another element, it can be directly connected to another element or there may be a central element at the same time. The terms "perpendicular", "horizontal", "left", "right" and similar expressions used herein are for illustrative purposes only, and are not meant to be the only implementation modes.

In addition, in the field of interventional medical instruments, an end closer to an operator is usually referred to as "proximal end", and an end farther from the operator is referred to as "distal end". The "proximal end" and the "distal end" of any component of a medical instrument are defined according to this principle. The "axial direction" usually refers to a longitudinal direction of a medical instrument during delivery; the "radial direction" usually refers to a direction perpendicular to the "axial direction" of the medical instrument. The "axial direction" and the "radial direction" of any component of a medical instrument are defined according to this principle.

In addition, the delivery device of the present disclosure is not only used for delivering a stent, but also used for delivering other implants similar to the stent. No limitations are made here. The technical solution of the embodiments will be further described in detail below in conjunction with specific embodiments by taking loading of a stent as an example.

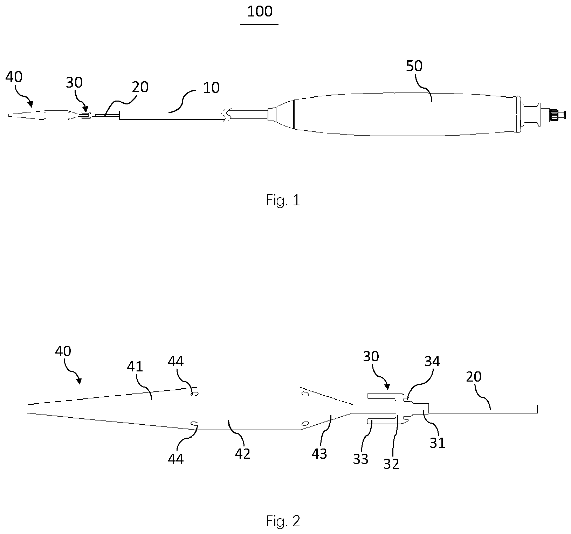

Referring to Fig. 1 and Fig. 2, the embodiments provide a delivery device 100. The delivery device 100 includes a sheath tube 10, a sheath core 20, a TIP head 40, a fixing anchor 30 and a handle 50. The sheath core 20 is threaded in an inner cavity of the sheath tube 10, and the sheath core 20 can move axially relative to the sheath tube 10. The TIP head 40 is arranged at a distal end of the sheath core 20, and a fixing anchor 30 is arranged on an outer wall of the sheath core 20. The fixing anchor is close to a proximal end of the TIP head 40 and is fixed relative to the TIP head 40. The fixing anchor 30 is provided with a mounting position 34, and a pull wire can be accommodated in the mounting position 34. The proximal end of the sheath tube 10 is connected with the handle 50. The relative movement of the sheath tube 10 and the sheath core 20 can be realized through the operation of the handle 50.

For example, the TIP head 40 is coaxially and fixedly connected to the distal end of the sheath core 20. The fixing anchor 30 is arranged on the sheath core 20 and fixed with the sheath core 20. The fixing anchor 30 is arranged close to the proximal end of the TIP head 40. When a stent is loaded, the proximal end of the stent is anchored to the fixing anchor 30, and then a pull wire 60 is threaded on the mounting position 34 of the fixing anchor 30. Both ends of the pull wire 60 are then threaded out of the distal end of the sheath tube 10 through the proximal end of the sheath tube 10. Both ends of the pull wire 60 passing through the distal end of the sheath tube 10 are then pulled to the distal end. Under the driving of the pull wire 60, the TIP head 40, the sheath core 20, the fixing anchor 30 and the stent enter the sheath tube 10 and move along an extending direction of the sheath tube 10. After the TIP head 40 protrudes from the distal end of the sheath tube 10 (only the TIP head 40 protrudes from the distal end of the sheath tube 10), the pull wire 60 is then separated from the fixing anchor 30 to load the stent into the delivery device 100.

During the process of loading the stent, the pull wire 60 applies an acting force on the fixing anchor 30, and the fixing anchor 30 is fixedly connected to the sheath core 20 and thus drives the sheath core 20, the TIP head 40 and the stent to move synchronously. Since the pull wire 60 does not directly act on the stent, the stent can be prevented from being deformed due to the pressing of the pull wire 60 on the stent. In addition, the stent is loaded by means of the pull wire 60, which prevents damage to the stent caused by pushing the sheath tube 10 of the prior art.

It should be pointed out that in the embodiments, the fixing anchor 30 surrounds the sheath core 20, and they are fixed by welding or bonding. The fixing anchor 30 and the sheath core 20 are connected and fixed by welding or bonding, which can effectively improve the strength of a junction between the fixing anchor 30 and the sheath core 20. When the pull wire is threaded on the mounting position 34 of the fixing anchor 30 to load the stent, the separation of the fixing anchor 30 from the sheath core 20 can be avoided, thus ensuring the loading effect of the stent.

In addition, the fixing anchor 30 is provided with a mounting position 34. When the stent is loaded, the proximal end of the stent is anchored to the fixing anchor 30. One end of the pull wire 60 passes through the mounting position 34 from the fixing anchor 30 and then extends to the distal end, so that both ends of the pull wire are located at the distal end of the mounting position 34. At the same time, both ends of the pull wire 60 are pulled to the distal end to achieve the loading of the stent. By means of the coordination between the pull wire 60 and the mounting position 34, the number of action positions of the pull wire 60 on the fixing anchor 30 is increased, so that the driving effect of the pull wire 60 on the fixing anchor 30 is improved, thereby improving the stent loading efficiency.

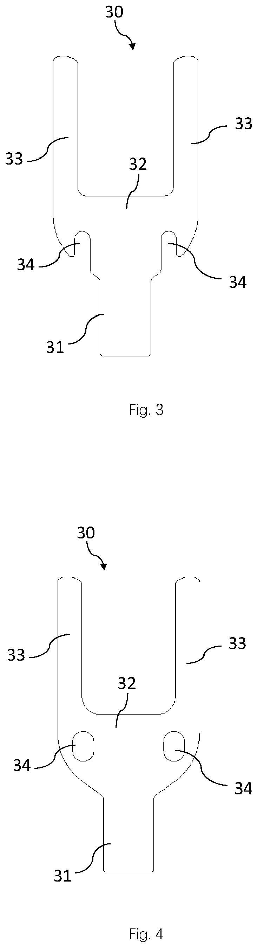

It is further understood that, as shown in Fig. 3, the fixing anchor 30 includes a fixing portion 31, an anchoring portion 33, and a basic portion 32. The fixing portion 31 surrounds the sheath core 20 and is fixed with the sheath core 20. The anchoring portion 33 is configured for anchoring the stent. The fixing portion 31 is connected with the anchoring portion 33 through the basic portion 32. The mounting position 34 is arranged on the basic portion 32 or the anchoring portion 33. The anchoring portion 33 includes a plurality of rod-shaped members extending from the proximal end of the basic portion 32 toward the distal end, and an accommodating space is formed between the plurality of rod-shaped members. In the embodiment, the fixing portion 31 is connected to the proximal end of the basic portion 32, and the anchoring portion 33 is connected to the distal end of the basic portion 32. The fixing portion 31 surrounds the sheath core 20 and is arranged close to the TIP head 40 (the TIP head 40 is located at the distal end of the sheath core 20). The fixing portion 31 and the sheath core 20 are connected and fixed by welding or bonding. The anchoring portion 33 extends to the distal end of the sheath core 20. When the stent is loaded, the pull wire 60 is in a threaded fit with the mounting position 34 on the fixing anchor 30. The pull wire 60 is pulled to the distal end, so that the TIP head 40, the stent and the sheath core 20 enter the sheath tube 10 until the TIP head 40 protrudes from an end of the sheath tube 10 to load the stent.

It should be understood that the radial size of the basic portion 32 is greater than the radial size of the fixing portion 31, and the radial size of the anchoring portion 33 is also greater than the radial size of the fixing portion 31. The mounting position 34 may be arranged on the basic portion 32 or the anchoring section 33.

It should be pointed out that the basic portion 32, the anchoring portion 33 and the fixing portion 31 are coaxially disposed, and are coaxial with the sheath core 20. When the pull wire 60 is threaded on the mounting position 34 of the fixing anchor 30 and drives the fixing anchor 30, an offset of the fixing anchor 30 can be effectively avoided, thus avoiding damage during the loading of the stent and ensuring the loading effect of the stent.

In some implementations, the basic portion 32, the anchoring portion 33 and the fixing portion 31 are of an integrated structure, and the fixing anchor 30 is formed by integrated molding. The connection strength between the various portions of this structure is high, and the stability is good, such that the deformation of the fixing anchor 30 does not affect the loading effect of the stent.

In some implementations, the basic portion 32, the anchoring portion 33 and the fixing portion 31 are of separate structures, and can be connected and fixed by welding or bonding. The fixing anchor 30 of this structure is convenient to machine. Compared with the integrated structure, the separate structures effectively reduce the manufacturing cost.

As shown in Fig. 1 to Fig. 3, in some implementations, the mounting position 34 includes a cutting slot formed in the basic portion 32, and an opening of the cutting slot faces the proximal end of the basic portion 32. For example, the cutting slot is formed in the basic portion 32, and the opening of the cutting slot faces the proximal end of the basic portion 32. When the pull wire 60 cooperates with the fixing anchor 30, the pull wire 60 extends to the distal end after passing through the cutting slot. The opening of the cutting slot is disposed at the proximal end of the basic portion 32. When the pull wire 60 moves to the distal end to drive the fixing anchor 30, a side wall of the cutting slot is configured for limiting the pull wire 60, thus avoiding the separation of the pull wire 60 from the fixing anchor 30, improving the stability of the pull wire 60 driving the fixing anchor 30, and ensuring the loading effect of the stent.

It should be pointed out that the depth of the cutting slot is greater than an outer diameter of the pull wire 60. When the pull wire 60 is threaded into the cutting slot, a side wall of the cutting slot is higher than the pull wire 60, which further improves the limiting effect on the pull wire 60, so as to prevent the pull wire 60 from falling out of the cutting slot.

In addition, when there is a plurality of mounting positions 34 for the cutting slot structure, the mounting positions 34 are disposed at equal intervals along a circumferential direction of the basic portion 32. When the pull wire 60 cooperates with each mounting position 34, it can be ensured that a force on the fixing anchor 30 is uniform and stable, so that the loading effect of the stent is further guaranteed.

It can be understood that in other embodiments, there can also be one cutting slot. At this time, the cutting slot is arranged around the basic portion 32 towards the proximal end.

In addition, the inner surface of the cutting slot is smooth, which reduces the friction between the cutting slot and the pull wire 60. After the stent is loaded, a resistance to the separation of the pull wire 60 from the cutting slot is reduced by means of reducing the friction.

As shown in Fig. 4, in some implementations, the mounting position 34 is a via hole formed in the basic portion 32. The via hole extends along a direction parallel to the radial direction of the sheath core 20 and extends through the basic portion 32 along this direction. For example, the via hole extends through the radial direction of the basic portion 32, and the two orifices of the via hole are respectively located on a peripheral surface of the basic portion 32. Two mounting positions 34 are taken for example. When the pull wire 60 cooperates with the fixing anchor 30, one end of the pull wire 60 is threaded out of the other end of the via hole through one end of one via hole. The pull wire 60 that comes out is then threaded out of the other end of the via hole through one end of another via hole. The pull wire 60 that comes out extends to the distal end. An external force simultaneously acts on both ends of the pull wire 60, and the pull wire 60 is pulled towards the distal end, thus loading the stent.

The mounting position 34 is a via hole. When the pull wire 60 is threaded in the via hole, the inside of the via hole can limit the pull wire 60, which improves the cooperation strength between the pull wire 60 and the fixing anchor 30, further avoids the separation of the pull wire 60 from the fixing anchor 30 when the pull wire 60 drives the fixing anchor 30, and ensures effective loading of the stent.

It should be pointed out that the diameter of the via hole is greater than the outer diameter of the pull wire 60. When the pull wire 60 is threaded in the via hole, it is convenient to separate the pull wire 60 from the fixing anchor 30 after the stent is loaded.

In addition, when there is a plurality of mounting positions 34 for the via hole structure, the mounting positions 34 are disposed at equal intervals along a circumferential direction of the basic portion 32. When the pull wire 60 cooperates with each mounting position 34, it can be ensured that a force on the fixing anchor 30 is uniform and stable, so that the loading effect of the stent is further guaranteed.

In addition, the orifice of the via hole is provided with a round chamfer, which reduces the friction between the orifice of the via hole and the pull wire 60, and also reduces the wear of the pull wire from the orifice. After the stent is loaded, the resistance to the separation of the pull wire 60 from the via hole is reduced by means of reducing the friction.

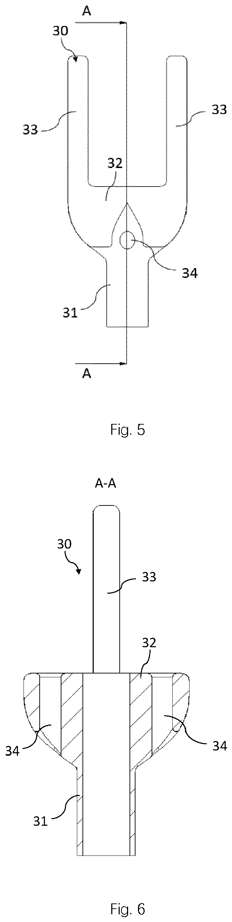

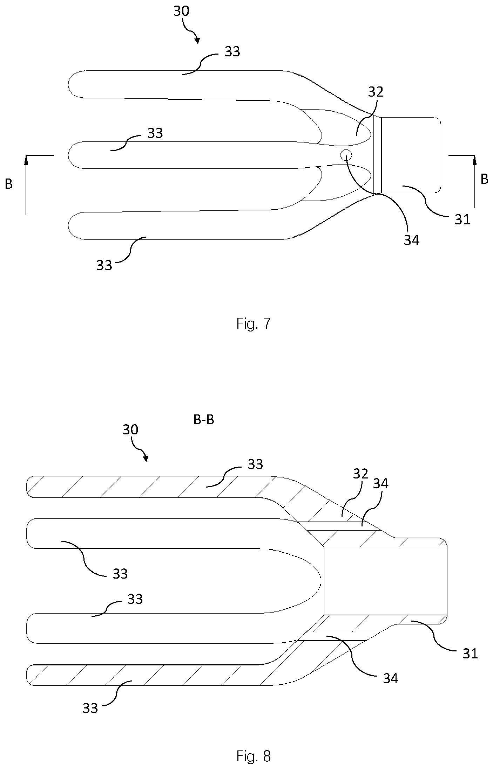

As shown in Fig. 5 to Fig. 8, in some implementations, the mounting position 34 is a through hole formed in the basic portion 32. The through hole extends along an axial direction of the sheath core 20. For example, the through hole extends through the distal end and the proximal end of the basic portion 32, and extends along the axial direction of the sheath core 20. Two mounting positions 34 are taken for example. When the pull wire 60 cooperates with the fixing anchor 30, one end of the pull wire 60 is threaded out of the other end of the through hole through one end of one through hole. The pull wire 60 that comes out is then threaded out of the other end of the through hole through the proximal end of another through hole. The pull wire 60 that comes out extends to the distal end. An external force simultaneously acts on both ends of the pull wire 60, and the pull wire 60 is pulled towards the distal end, thus loading the stent.

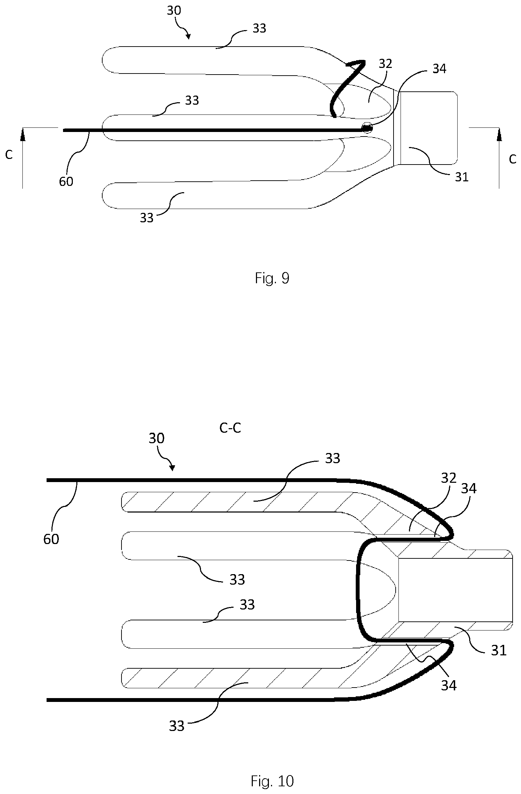

As shown in Fig. 9 to Fig. 11, the mounting position 34 is a through hole. When the pull wire 60 is threaded in the through hole, the inside of the through hole can limit the pull wire 60, which improves the cooperation strength between the pull wire 60 and the fixing anchor 30, further avoids their separation when the pull wire 60 drives the fixing anchor 30, and ensures effective loading of the stent.

It should be pointed out that the diameter of the through hole is greater than the outer diameter of the pull wire 60. When the pull wire 60 is threaded in the through hole, the side wall of the through hole is spaced apart from the pull wire 60. It is convenient to separate the pull wire 60 from the fixing anchor 30 after the stent is loaded.

In addition, when there is a plurality of mounting positions 34 for the through hole structure, the mounting positions 34 are disposed at equal intervals along a circumferential direction of the basic portion 32. When the pull wire 60 cooperates with each mounting position 34, it can be ensured that a force on the fixing anchor 30 is uniform and stable, so that the loading effect of the stent is further guaranteed.

In addition, the orifice of the through hole is provided with a round chamfer, which reduces the friction between the orifice of the through hole and the pull wire 60, and also reduces the wear of the pull wire from the orifice. After the stent is loaded, the resistance to the separation of the pull wire 60 from the through hole is reduced by means of reducing the friction.

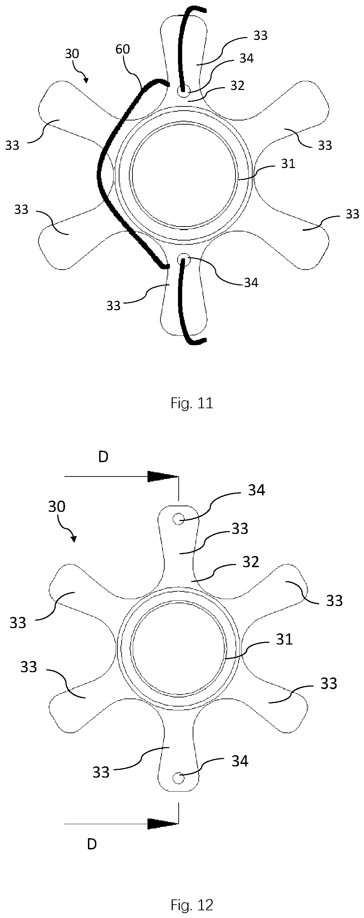

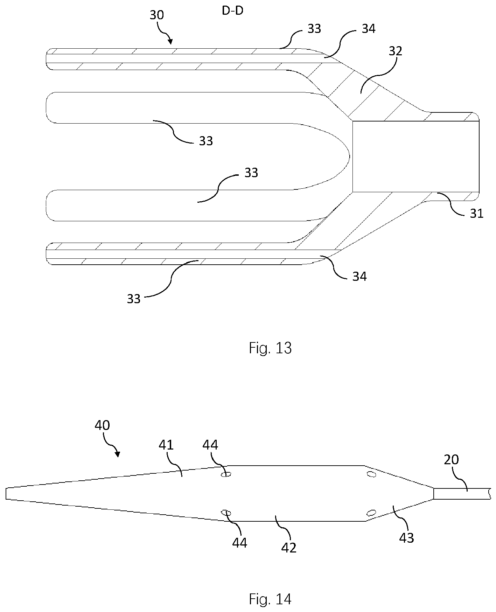

Further, as shown in Fig. 12 and Fig. 13, the anchoring portion 33 includes a plurality of rod-shaped members, each of which is arranged at the distal end of the basic portion 32. Each rod-shaped member extends towards the distal end. The mounting position 34 includes punch holes arranged on at least some of the rod-shaped members, and the punch holes extend along the axial direction of the sheath core 20. For example, the rod-shaped members are connected to the distal end of the basic portion 32, and are arranged at equal intervals along the circumferential direction of the basic portion 32. An accommodating space is formed among the plurality of rod-shaped members. The rod-shaped members are configured for anchoring the proximal end of the stent, and limiting a proximal corrugated ring portion of the stent to the accommodating space.

The punch hole runs through the distal end and the proximal end of the rod-shaped member, and extends along the axial direction of the sheath core 20. Two mounting positions 34 are taken for example. When the pull wire 60 cooperates with the fixing anchor 30, one end of the pull wire 60 is threaded out of the other end of the punch hole through the distal end of one punch hole. The pull wire 60 that comes out bypasses an outer surface of the basic portion 32 to the rod-shaped member of the next punch hole, and is then threaded out of the proximal end of the punch hole through the proximal end of another punch hole. The pull wire 60 that comes out extends to the distal end. An external force simultaneously acts on both ends of the pull wire 60, and the pull wire 60 is pulled towards the distal end, thus loading the stent.

The mounting position 34 is a punch hole. When the pull wire 60 is threaded in the punch hole, the inside of the punch hole can limit the pull wire 60, which improves the cooperation strength between the pull wire 60 and the fixing anchor 30, further avoids their separation when the pull wire 60 drives the fixing anchor 30, and ensures effective loading of the stent. In addition, the pull wire 60 is threaded on the rod-shaped member, which further improves the fixing strength and stability of the pull wire 60 and the fixing anchor 30, and further enhances the loading effect of the stent.

It should be pointed out that the diameter of the punch hole is greater than the outer diameter of the pull wire 60. When the pull wire 60 is threaded in the punch hole, the side wall of the punch hole is spaced apart from the pull wire 60. It is convenient to separate the pull wire 60 from the fixing anchor 30 after the stent is loaded.

In addition, the orifice of the punch hole is provided with a round chamfer, which reduces the friction between the orifice of the punch hole and the pull wire 60, and also reduces the wear of the pull wire from the orifice. After the stent is loaded, the resistance to the separation of the pull wire 60 from the punch hole is reduced by means of reducing the friction.

Further, there are two punch holes, and two rod-shaped members with the punch holes are opposite, adjacent or spaced apart. For example, the mounting positions of the punch hole structure are arranged on the rod-shaped members. When there are two punch holes, the two punch holes are respectively arranged on the adjacent, opposite or spaced rod-shaped members. When the pull wire 60 cooperates with the fixing anchor 30, the stability of the action on the anchoring portion 33 is further improved, and the loading effect of the stent is further enhanced.

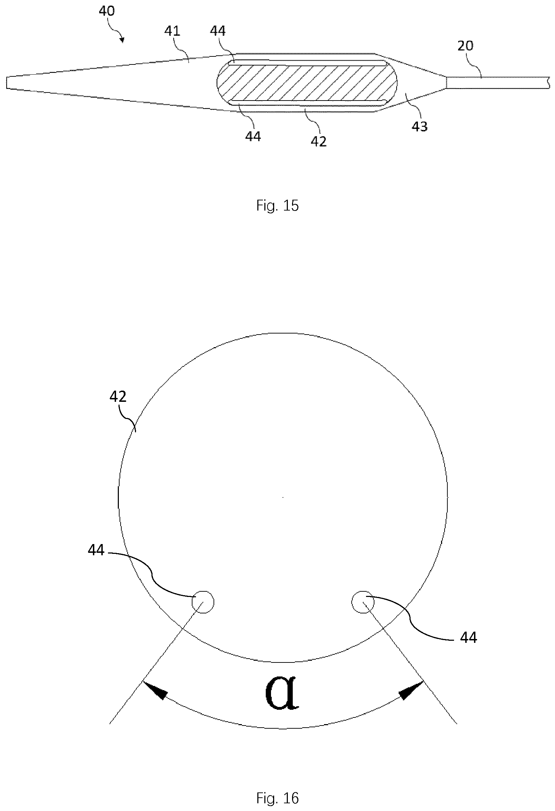

As shown in Fig. 14 and Fig. 15, the TIP head 40 includes a first tapered section 41, a second tapered section 43 and a cylindrical section 42. The first tapered section 41 is coaxially arranged at a distal end of the cylindrical section 42. The second tapered section 43 is coaxially arranged at a proximal end of the cylindrical section 42 and is connected with the sheath core 20. A fixing position 44 is arranged on the cylindrical section 42. The fixing position 44 is disposed along a longitudinal direction of the cylindrical section 42, and the pull wire can be accommodated in the fixing position 44. When the stent is loaded, one end of the pull wire 60 passes through the mounting position 34 of the fixing anchor 30 in turn, and the pull wire 60 that comes out passes through the fixing position 44 on the cylindrical section 42 and then extends to the distal end. By means of pulling the pull wire 60 to the distal end, the stent is loaded into the sheath tube 10 to load the stent.

By means of setting the fixing position 44 on the TIP head 40, the pull wire 60 can be prevented from being deviated or twisted, and can also be prevented from protruding from the TIP head, so as to avoid the following phenomenon: The volume of the TIP head increases due to the pull wire, so that the sheath tube or the TIP head and the internal and external sheath cores are pressed to be damaged or deformed.

It should be pointed out that the taper of the first tapered section 41 is less than that of the second tapered section 43, thereby making it easier for the TIP head 40 to enter and leave the sheath tube 10.

It can be understood that in other embodiments, the TIP head may not include the first tapered section and/or the second tapered section.

In one embodiment, the fixing position 44 includes a via hole which is arranged along a longitudinal direction of the cylindrical section 42 and axially extends through the cylindrical section 42. A port of the distal end of the via hole is located at a junction between the first tapered section 41 and the cylindrical section 42, and a port of the proximal end of the via hole is located at a junction between the second tapered section 43 and the cylindrical section 42. Two mounting positions 34 and two fixing positions 44 are taken for example where the mounting positions 34 are through holes extending through the distal end and the proximal end of the basic portion 32. When the stent is loaded, after the proximal end of the stent is anchored to the fixing anchor 30, one end of the pull wire 60 is threaded out of the proximal end of the fixing position through the distal end of one fixing position, and is then threaded out of the proximal end of the mounting position through the proximal end of one mounting position. The pull wire 60 that comes out bypasses the basic portion, and is then threaded out of the distal end of the mounting position through the proximal end of the other mounting position. The pull wire 60 that comes out is threaded out of the distal end of the fixing position through the proximal end of the other fixing position and then extends to the distal end. The pull wire 60 is pulled to the distal end, so that the stent is loaded in the sheath tube 10, thus achieving the loading of the stent. It can be understood that the mounting sequence of the pull wire is not limited to the above sequence, as long as the pull wire finally passes through the mounting positions and fixing positions, and both ends of the pull wire are located at both ends of the tip head.

Since the via holes are formed inside the cylindrical section 42, when the pull wire 60 passes through the via holes, the pull wire 60 will not increase the radial size of the cylindrical section 42 of the TIP head 40, which then will not cause a loading difficulty.

In addition, the orifice of the via hole is provided with a round chamfer, which reduces the friction between the orifice of the via hole and the pull wire 60, and also reduces the wear of the pull wire from the orifice. After the stent is loaded, the resistance to the separation of the pull wire 60 from the via hole is reduced by means of reducing the friction.

As shown in Fig. 16, the fixing positions 44 are arranged on the cylindrical section 42 of the TIP head 40. The two fixing positions 44 are arranged at intervals along the circumferential direction of the cylindrical section 42. An angle of circumference formed by the two fixing positions 44 on the same section of the cylindrical section 42 is a, which ranges from 0° to 180°, thus improving the convenience of the layout of the pull wire 60. The through hole on the TIP head for allowing a guide wire to pass is omitted in Fig. 16.

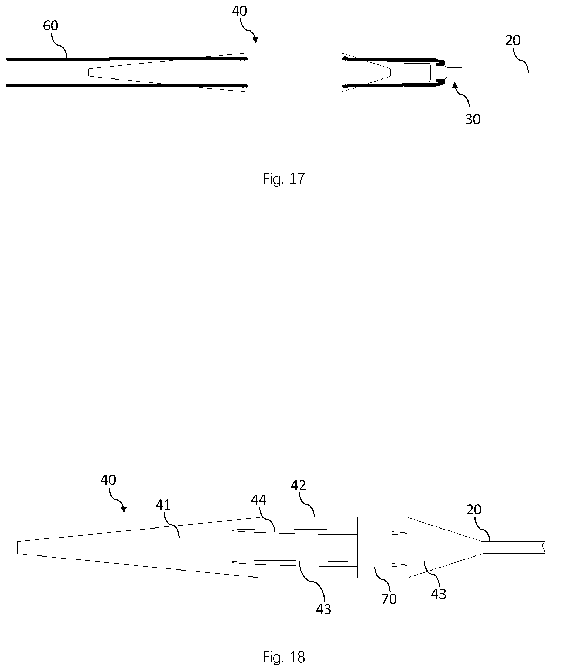

As shown in Fig. 17, there are two fixing positions 44 and two mounting positions 34. A connecting line of the proximal ends of the two fixing positions 44 is parallel to a connecting line of the two mounting positions 34. For example, when there are two fixing positions 44 and two mounting positions 34, the connecting line of the proximal ends of the two fixing positions 44 is kept being parallel to the connecting line of the two mounting positions 34, thus avoiding the torsion of the pull wire 60, ensuring the stability of the pull wire 60 driving the fixing anchor 30, and also ensuring the effective separation of the pull wire 60 from the fixing anchor 30.

Since the through slot is formed inside the cylindrical section 42, when the pull wire 60 passes through the through slot, the pull wire 60 will not increase the radial size of the cylindrical section 42 of the TIP head 40, which further avoids the impact of the pull wire 60 on the stent and ensures the loading effect of the stent.

In addition, the inner surface of the through slot is smooth, which reduces the friction between the through slot and the pull wire 60. After the stent is loaded, the resistance to the separation of the pull wire 60 from the through slot is reduced by means of reducing the friction.

In other embodiments, as shown in Fig. 18, the fixing position can also be a groove formed on a surface of the cylindrical section 42. The groove extends along a longitudinal direction of the cylindrical section 42, and an opening is located on the outer surface of the cylindrical section. A distal end of the groove is located at the junction between the first tapered section and the cylindrical section, and a proximal end of the groove is located at the junction between the second tapered section and the cylindrical section. In order to further fix the pull wire, the delivery device 100 also includes a fixing member 70. The fixing member 70 surrounds the proximal end of the groove to fix the pull wire. It can be understood that in order to reduce the impact of the fixing member 70 on the overall size of the TIP head 40, a circumferential groove can be arranged at a position on the cylindrical section of the TIP head 40 where the fixing member 70 is provided, which can exactly accommodate the fixing member 70. At this time, the inner surface of the fixing member 70 can also be correspondingly provided with a groove for accommodating the pull wire.

It should be understood that an outer surface of the fixing member 70 is flush with the outer surface of the cylindrical section 42, avoiding the impact of the fixing member 70 on the stent, further ensuring that the stent is not damaged, and enhancing the loading effect of the stent.

In summary, implementing the delivery device of the present embodiments have the following beneficial effects:
1. The mounting position and mounting hole are arranged on the fixing anchor and the TIP head to avoid the pull wire from protruding, so as to avoid the following phenomenon: The volume of the TIP head increases due to the pull wire, so that the sheath tube or the TIP head and the internal and external sheath cores are pressed to be damaged or deformed.
2. The mounting position is arranged on the fixing anchor, which can avoid the deformation of the sheath core and make stent assembly easier compared with the direct pushing of the stent hooked with the sheath core. In addition, a pulling force acts on the delivery device to avoid damage to the stent caused by directly perforating the stent. The stent will not move during the assembly.
3. By means of cooperation between a pull wire hole or slot and other pull wire fixing members, the pull wire is stable, will not damage and affect the stent and the sheath tube of the delivery device, and can be withdrawn smoothly.

Each feature of the above-described embodiments can be combined in any combination, and in order to make the description concise, not all the possible combinations of each feature in the above-described embodiments are described. However, as long as there is no contradiction between the combinations of these features, they should be considered as the scope of the embodiments.

The above- described embodiments only express several implementations, which are described in greater detail but are not to be construed as limiting the scope of the embodiments. It should be noted that those of ordinary skill in the art can further make various transformations and improvements without departing from the concept of the embodiments, and these transformations and improvements all fall within the protection scope of the embodiments. Therefore, the protection scope of the patent of the embodiments shall be subject to the appended claims.

## Claims

1. A delivery device, comprising:
a sheath core;
a TIP head, the TIP head arranged at a distal end of the sheath core; and
a fixing anchor, wherein the fixing anchor is arranged on an outer wall of the sheath core and close to a proximal end of the TIP head; the sheath core is fixed relative to the TIP head; the fixing anchor is provided with a mounting portion, and a pull wire is accommodated in the mounting portion.

2. The delivery device according to claim 1, wherein the fixing anchor comprises:
a fixing portion, which is arranged on an outer side of the sheath core and is fixed with the sheath core;
an anchoring portion, which is configured for anchoring a stent; and
a basic portion, wherein the fixing portion is connected with the anchoring portion through the basic portion, and the mounting position is arranged on the basic portion or the anchoring portion.

3. The delivery device according to claim 2, wherein the mounting position comprises a cutting slot formed in the basic portion, and an opening of the cutting slot faces a proximal end of the basic portion;
or the mounting position comprises a through hole formed in the basic portion, and the through hole extends along an axial direction of the sheath core and axially extends through the basic portion;
or the mounting position comprises a via hole formed in the basic portion, and the via hole extends along a direction parallel to a radial direction of the sheath core and extends through the basic portion along the direction.

4. The delivery device according to claim 2, wherein the anchoring portion comprises a plurality of rod-shaped members; the rod-shaped members are arranged at a distal end of the basic portion and extend towards the distal end; the mounting position comprises punch holes formed in the rod-shaped members; and the punch holes extend along an axial direction of the sheath core.

5. The delivery device according to claim 4, wherein two punch holes are formed, and are arranged on opposite, adjacent or spaced rod-shaped members.

6. The delivery device according to any one of claims 1 to 5, wherein the TIP head comprises a cylindrical section; the cylindrical section is provided with a fixing position arranged along a longitudinal direction of the cylindrical section; and the pull wire is accommodated in the fixing position.

7. The delivery device according to claim 6, wherein the TIP head further comprises a first tapered section and a second tapered section; the first tapered section is coaxially arranged at a distal end of the cylindrical section; the second tapered section is coaxially arranged at a proximal end of the cylindrical section and is connected with the sheath core; the fixing position comprises a via hole which is arranged along a longitudinal direction of the cylindrical section and axially extends through the cylindrical section; a port of a distal end of the via hole is located at a junction between the first tapered section and the cylindrical section; and a port of a proximal end of the via hole is located at a junction between the second tapered section and the cylindrical section.

8. The delivery device according to claim 6, wherein the TIP head further comprises a first tapered section and a second tapered section; the first tapered section is coaxially arranged at a distal end of the cylindrical section; the second tapered section is coaxially arranged at a proximal end of the cylindrical section and is connected with the sheath core; the fixing position comprises a groove which is arranged along a longitudinal direction of the cylindrical section; an opening of the groove is located on an outer surface of the cylindrical section; a distal end of the groove is located at a junction between the first tapered section and the cylindrical section; and a proximal end of the groove is located at a junction between the second tapered section and the cylindrical section.

9. The delivery device according to claim 8, wherein the delivery device further comprises a fixing member, which surrounds a proximal end portion of the groove to fix a pull wire.

10. The delivery device according to claim 9, wherein a circumferential groove is arranged at a proximal end portion of the cylindrical section; and the fixing member is accommodated in the circumferential groove, so that an outer surface of the fixing member is flush with an outer surface of the cylindrical section.
